# EUROPÄISCHE PATENTSCHRIFT

(11) **EP 3 355 961 B1**
(45) Veröffentlichungstag und Bekanntmachung des Hinweises auf die Patenterteilung: **11.12.2024**
(21) Anmeldenummer: 16795224.1
(22) Anmeldetag: 19.09.2016
(51) Int. Cl.: A61M 5/178, A61J 1/20

(54) **FÜLLHILFE**
FILLING AID
AIDE DE REMPLISSAGE

(30) Priorität: 29.09.2015 DE 102015218723
(43) Veröffentlichungstag der Anmeldung: 08.08.2018
(73) Patentinhaber: Transcoject GmbH, 24539 Neumünster (DE)
(72) Erfinder: WALLMANN, Stefan, 22763 Hamburg (DE); HEINZ, Jochen, 24220 Flintbek (DE); SCHILLING, Dieter, 24613 Aukrug (DE)
(74) Vertreter: Patentanwälte Hemmer Lindfeld Frese Partnerschaft mbB
(86) Internationale Anmeldenummer: PCT/DE2016/200437
(87) Internationale Veröffentlichungsnummer: WO 2017/054812

(56) Entgegenhaltungen:
- EP-A1- 1 757 320
- WO-A1-2005/011781
- WO-A1-2011/109915
- US-A- 4 338 980
- US-A- 4 434 820
- US-A- 4 475 915
- US-A1- 2012 055 580

## Beschreibung

Die Erfindung betrifft eine Füllhilfe zum Befüllen einer Zylinderampulle sowie ein Verfahren zum Befüllen einer Zylinderampulle unter Verwendung einer derartigen Füllhilfe.

In verschiedenen medizinischen Anwendungen ist es erforderlich, eine medizinische oder pharmazeutische Flüssigkeit aus einem Behältnis in eine Zylinderampulle, eine sogenannte Karpule, zu transferieren. Dies ist insbesondere dann der Fall, wenn die genannte Flüssigkeit zuvor aus einem trockenen, insbesondere pulverförmig vorliegenden medizinischen oder pharmazeutischen Stoff durch Zuführen eines flüssigen Lösungsmittels, beispielsweise von Kochsalzlösung oder Wasser für Injektionszwecke, aufgelöst werden muss.

Solche Zylinderampullen oder Karpulen kommen insbesondere in sogenannten "Pen-Systemen" oder Karpulen-Spritzen zum Einsatz, welche auch zum Heimgebrauch vorgesehen sein können. Solche Pen-Systeme sind insbesondere für Insulin bekannt, können jedoch auch für andere medizinische oder pharmazeutische Wirkstoffe Verwendung finden, beispielsweise für Hormonpräparate für Kinder und Jugendliche. Diese liegen in der Regel in Pulverform vor und müssen zunächst in der oben beschriebenen Weise aufgelöst und dann in eine Zylinderampulle bzw. Karpule transferiert werden, um diese dann in die Karpulen-Spritze bzw. das Pen-System einzusetzen. Dies ist gerade für einen medizinischen Laien nicht ganz einfach. Insbesondere ist es problematisch, bei diesen Vorgängen die erforderliche Sterilität aufrechtzuerhalten.

EP 1 757 320 A1 offenbart eine Vorrichtung, die zum Befüllen eines Pen-Systems zum Applizieren eines flüssigen Medikamentes dient. Die Vorrichtung ist geeignet, ein pulverförmiges Medikament mit einer Flüssigkeit zu mischen und dann in das Pen-System dosiert zu applizieren. Hierzu ist in der Vorrichtung eine aufwendige Mechanik vorgesehen. Darüber hinaus sind austauschbare Kassetten vorgesehen, welche jeweils einen Behälter mit dem pulverförmigen Medikament und einen Behälter mit der Flüssigkeit sowie eine Aufnahme für gebrauchte Spritzen enthalten. Dies führt zu einem insgesamt recht großen und teuren Aufbau der Vorrichtung.

WO 2011/109915 A1 offenbart eine modulare Befüllvorrichtung für einen Applikator, bei welchem verschiedene Behältnisse und Spritzen über einen Applikatorhalter mit dem Applikatorkörper zu dessen Befüllen verbunden werden. Hierbei müssten verschiedene Komponenten in richtiger Weise miteinander verbunden werden, sodass für einen Laien das Risiko von Fehlbedienungen besteht.

US 4,434,820 offenbart eine Vorrichtung zum Befüllen von Spritzen mit einer Halterung, in welcher zwei Behälter für Flüssigkeiten angeordnet sind. Ferner ist eine Halterung für eine Spritze zu deren Befüllen aus den Behältern vorgesehen, wobei Begrenzungshebel zum Begrenzen des Kolbenhubes der Spritze zum Festlegen einer definierten Dosierung vorgesehen sind.

US 4,338,980, WO 2005/011781 A1 sowie US 4,475,915 offenbaren Halterungen, in welchen ein Behältnis mit einer Flüssigkeit und eine Spritze gehalten werden können, um die Flüssigkeit aus dem Behältnis in die Spritze zu transferieren. Diese Vorrichtungen sind nicht speziell dafür vorgesehen, pulverförmige Medikamente oder Arzneimittel aufzulösen und in eine Zylinderampulle bzw. Kapule zu transferieren.

Es ist im Hinblick auf diese Problematik Aufgabe der Erfindung, eine Füllhilfe zum Befüllen einer Zylinderampulle bereitzustellen, welche es ermöglicht, auf einfache Weise eine medizinische oder pharmazeutische Flüssigkeit aus einem Behältnis in eine Zylinderampulle bzw. Karpule zu transferieren.

Diese Aufgabe wird durch eine Füllhilfe mit den in Anspruch 1 angegebenen Merkmalen sowie durch ein Verfahren mit den in Anspruch 15 angegebenen Merkmalen gelöst. Bevorzugte Ausführungsformen ergeben sich aus den Unteransprüchen, der nachfolgenden Beschreibung sowie den beigefügten Figuren.

Die erfindungsgemäße Füllhilfe dient dazu, eine medizinische oder pharmazeutische Flüssigkeit aus einem Behältnis, in welchem diese Flüssigkeit bereitgestellt oder angemischt wird, in eine Zylinderampulle zu transferieren. Diese Füllhilfe kann für verschiedenste medizinische oder pharmazeutische Flüssigkeiten Verwendung finden.

Die erfindungsgemäße Füllhilfe weist als wesentliches Element zumindest eine Haltehilfe auf. Diese weist ein Gehäuse auf, in welchem eine erste Aufnahme ausgebildet ist. Diese erste Aufnahme ist dafür ausgebildet, die zu befüllende Zylinderampulle aufzunehmen. Das bedeutet, die Aufnahme ist so dimensioniert und geformt, dass eine zu befüllende Zylinderampulle, bei welcher es sich bevorzugt um eine Zylinderampulle für übliche Zylinderampullenspritzen handelt, aufgenommen werden kann. Derartige Zylinderampullen sind am Markt unter der Bezeichnung Karpule bekannt. Ferner ist in dem Gehäuse eine zweite Aufnahme vorgesehen, welche dafür ausgebildet ist, ein Behältnis aufzunehmen, in welchem die medizinische oder pharmazeutische Flüssigkeit bereitgestellt wird. Das bedeutet die zweite Aufnahme ist so dimensioniert und geformt, dass in sie ein übliches Behältnis zur Aufnahme eines medizinischen oder pharmazeutischen Stoffes eingesetzt und aufgenommen werden kann. Bei diesem Behältnis handelt es sich insbesondere um ein übliches Vial. Anstatt in dem Behältnis die medizinische oder pharmazeutische Flüssigkeit in fertiger Form bereitzustellen, kann das Behältnis auch einen medizinischen oder pharmazeutischen Stoff aufweisen, welcher zunächst mit einem zuzugebenden Lösungsmittel, beispielsweise Kochsalzlösung oder Wasser aufgelöst werden muss, um so die medizinische oder pharmazeutische Flüssigkeit zu erzeugen. Dabei erfolgt dieses Auflösen vorzugsweise in der Füllhilfe, d. h. in einem Zustand, in welchem das Behältnis in der zweiten Aufnahme aufgenommen ist.

An der ersten Aufnahme ist eine erste Zugangsöffnung ausgebildet. Diese ist so angeordnet und ausgebildet, dass sie einer Öffnung einer Zylinderampulle, wenn diese in die erste Aufnahme eingesetzt ist, gegenüberliegt. D. h. durch die erste Zugangsöffnung ist die Öffnung bzw. ein diese Öffnung verschließendes Septum einer eingesetzten Zylinderampulle von außen zugänglich, sodass durch die erste Zugangsöffnung hindurch die Zylinderampulle, welche in dem Gehäuse der Füllhilfe angeordnet ist, befüllt werden kann. Ferner weist das Gehäuse eine zweite Zugangsöffnung auf, welche an der zweiten Aufnahme angeordnet ist. Diese zweite Zugangsöffnung ist so ausgebildet und so gelegen, dass sie einer Öffnung bzw. einem die Öffnung verschließenden Verschluss eines Behältnisses, wenn dieses in die zweite Aufnahme eingesetzt ist, gegenüberliegt. D. h. die Öffnung bzw. der Verschluss (z. B. ein Septum) des Behältnisses ist, wenn dieses in die zweite Aufnahme eingesetzt ist, durch die zweite Zugangsöffnung zugänglich, sodass durch die zweite Zugangsöffnung hindurch beispielsweise ein Lösungsmittel zum Auflösen eines Pulvers in das Behältnis eingebracht werden kann und/oder der Inhalt des Behältnisses aus diesem entnommen werden kann.

Diese Ausgestaltung der erfindungsgemäßen Füllhilfe hat den Vorteil, dass in einem Bauteil, nämlich der Haltehilfe, die beiden Elemente, zwischen denen die medizinische oder pharmazeutische Flüssigkeit zu transferieren ist, sicher gehalten werden können. So kann die Füllhilfe gleichzeitig das Behältnis, in welchem die Flüssigkeit angemischt oder bereitgestellt wird und die zu befüllende Zylinderampulle halten. Dadurch wird die Handhabung vereinfacht, da der Transfer der Flüssigkeit von dem Behältnis in die Zylinderampulle dann beispielsweise mithilfe einer Spritze erfolgen kann, welche zuerst in die zweite Zugangsöffnung eingesetzt wird, um den Inhalt des Behältnisses in die Spritze aufzunehmen. Anschließend kann die Spritze von der zweiten Zugangsöffnung in die erste Zugangsöffnung umgesteckt werden und dann der Inhalt aus der Spritze in die Zylinderampulle transferiert werden. Die Füllhilfe bzw. deren Haltehilfe hält dabei die Zylinderampulle und das Behältnis insbesondere so, dass deren Öffnungen geschützt sind und Verunreinigungen verhindert werden. Nach dem Transfer der Flüssigkeit in die Zylinderampulle, kann diese aus der ersten Aufnahme entnommen, beispielsweise in eine Karpulenspritze oder ein herkömmliches Pen-System zur Verwendung eingesetzt werden. Eine solche Gestaltung ist nicht nur für die Anwendung durch den Patienten selber von Vorteil, sondern auch bei der Anwendung z. B. in Kliniken, Arztpraxen und Laboren.

Das Gehäuse der erfindungsgemäßen Füllhilfe ist vorzugweise aus Kunststoff und weiter bevorzugt als einteiliges und insbesondere einstückiges Kunststoffbauteil ausgebildet. Insbesondere kann das Gehäuse als Spritzgussteil aus Kunststoff gefertigt sein, was eine kostengünstige Herstellung ermöglicht.

In der ersten Aufnahme sind vorzugsweise Fixiermittel angeordnet, welche dazu ausgebildet und vorgesehen sind, eine Zylinderampulle in der Aufnahme lösbar zu fixieren. Die Fixiermittel sind vorzugsweise zum Halten einer üblichen, d. h. genormten Zylinderampulle bzw. Karpule ausgebildet. Die Fixiermittel sind so ausgebildet und angeordnet, dass sie die Zylinderampulle in der Aufnahme festhalten, wenn durch die erste Zugangsöffnung eine Spritze eingesetzt wird, um die Zylinderampulle zu befüllen. Ferner sind die Fixiermittel jedoch so ausgebildet, dass die Zylinderampulle aus der Aufnahme entnommen werden kann. Dazu kann die Zylinderampulle ergriffen werden und auf die Zylinderampulle eine Kraft ausgeübt werden, welche eine Haltekraft der Fixiermittel übersteigt. Die Entnahmerichtung der Zylinderampulle aus der ersten Aufnahme erstreckt sich dabei vorzugsweise in Längsrichtung der Zylinderampulle. Dies ist weiter bevorzugt gleichzeitig auch die Einsetzrichtung, in welcher eine Spritze in die erste Zugangsöffnung eingesetzt werden kann, um mit der Öffnung der Zylinderampulle in Verbindung zu treten, um eine Flüssigkeit aus der Spritze in die Zylinderampulle einzubringen. Die Fixiermittel sind vorzugsweise als kraft- und/oder formschlüssige Fixierelemente ausgebildet, welche vorzugsweise die Zylinderampulle an ihrem vorderen, d. h. an die Öffnung angrenzenden Ende ergreift. Weiter bevorzugt können die Fixiermittel als Rasthaken bzw. Rastzungen ausgebildet sein. Insbesondere können die Rasthaken bzw. Rastzungen einen die Öffnung umgebenden Kragen um- bzw. hintergreifen. Dabei sind die Rasthaken bzw. Rastzungen vorzugsweise so angeordnet, dass sie bei einer ausreichend großen Krafteinwirkung in Längsrichtung der Zylinderampulle aus ihrer Rastposition ausgelenkt werden und die Zylinderampulle freigeben. Derartige Rastzungen bzw. Rasthaken sind vorzugsweise einteilig, weiter bzw. einstückig mit zumindest einem Teil des Gehäuses, vorzugsweise mit dem gesamten Teil des Gehäuses, ausgebildet. Insbesondere bei Fertigung des Gehäuses aus Kunststoff können derartige Rasthaken oder Rastzungen auf einfache Weise mit ausgebildet werden.

Gemäß einer besonders bevorzugten Ausführungsform ist die Füllhilfe so ausgebildet, dass eine Zylinderampulle in der ersten Aufnahme mit den Fixiermitteln vormontiert und lösbar fixiert ist, sowie aus der ersten Aufnahme entnehmbar ist. So wird die Füllhilfe dem Nutzer in diesem vormontierten Zustand ausgeliefert, sodass der Nutzer die Zylinderampulle nicht mehr in die Füllhilfe einzusetzen braucht. Dies hat den Vorteil, dass die Handhabung weiter vereinfacht wird und die Zylinderampulle sicher und geschützt in der Füllhilfe fixiert ist.

Gemäß einer weiteren bevorzugten Ausführungsform sind in der zweiten Aufnahme Fixiermittel angeordnet, welche dazu ausgebildet und vorgesehen sind, ein einen medizinischen oder pharmazeutischen Stoff enthaltendes Behältnis in der Aufnahme bevorzugt lösbar zu fixieren. Bei einem solchen Behältnis kann es sich um ein im medizinischen Bereich übliches Vial handeln. D. h. die Fixiermittel sind vorzugsweise so dimensioniert und ausgebildet, dass sie ein solches Vial fixieren können. Die Fixierung durch die Fixiermittel ist vorzugsweise in der Art lösbar, dass durch ausreichende Krafteinwirkung das Behältnis aus der Aufnahme unter Lösen der Fixierung entnehmbar ist. Dabei ist die Entnahmerichtung vorzugsweise so gelegen, dass sie der Längs- bzw. Mittelachse des Behältnisses entspricht, welche vorzugsweise ebenfalls der Einsetzrichtung einser Spritze in die zweite Zugangsöffnung entspricht. Die Fixiermittel sind vorzugsweise als Rasthaken oder Rastzungen ausgebildet, welche das Behältnis an einem Kragen hintergreifen. Ein derartiger Kragen ist bei den üblichen Behältnissen an demjenigen Axialende, an welchem die Öffnung gelegen ist, ausgebildet und umgibt und/oder hält den Verschluss des Behältnisses. Die Rastzungen bzw. Rasthaken sind vorzugsweise einstückig bzw. einteilig mit zumindest einem Teil des Gehäuses, vorzugsweise mit dem gesamten Gehäuse ausgebildet. So können diese Rasthaken kostengünstig im Spritzguss gemeinsam mit dem Gehäuse gefertigt werden. Die Rasthaken bzw. Rastzungen sind vorzugsweise so ausgebildet, dass sie sich bei ausreichend großer Krafteinwirkung auf das Behältnis aus ihrer Verriegelungsposition in eine gelöste Position bewegen, sodass das Behältnis aus der Aufnahme entnommen werden kann. Es ist weiter bevorzugt jedoch auch möglich, das Behältnis mit dem medizinischen oder pharmazeutischen Stoff oder der medizinischen oder pharmazeutischen Flüssigkeit separat auszuliefern, sodass es vom Nutzer in die zweite Aufnahme zunächst eingesetzt werden muss und dort vorzugsweise mit den Fixiermitteln verrastet und/oder verklemmt wird. Nachdem die medizinische oder pharmazeutische Flüssigkeit in der oben beschriebenen Weise aus dem Behältnis entnommen worden ist, kann das Behältnis in der Aufnahme verbleiben, da es nicht weiter benötigt wird. Alternativ kann es beispielsweise zur getrennten Entsorgung auch vom Nutzer aus der Aufnahme wieder entnehmbar sein, sofern die Fixiermittel in der zweiten Aufnahme zu einer lösbaren Fixierung ausgebildet sind.

Gemäß einer weiteren bevorzugten Ausführungsform ist in der zweiten Aufnahme ein Kanülenelement lösbar gehalten. Dieses Kanülenelement weist eine Kanüle und einen Luer-Anschluss auf. Dabei ist das Kanülenelement in der zweiten Aufnahme so angeordnet, dass der Luer-Anschluss der Kanüle der zweiten Zugangsöffnung zugewandt ist und die Kanüle so gerichtet ist, dass sie in ein in die zweite Aufnahme eingesetztes Behältnis eindringt. Das Kanülenelement dient dazu, mit seiner Kanüle in die Öffnung des Behältnisses einzudringen und insbesondere eine Dichtung, beispielsweise ein Septum, in der Öffnung des Behältnisses zu durchstechen.

Besonders bevorzugt ist das Kanülenelement in der zweiten Aufnahme in einer Linearführung angeordnet, welche eine Bewegung des Kanülenelementes in seiner Längsrichtung, d. h. in Längsrichtung der Kanüle, ermöglicht. Dabei ist das Kanülenelement in der Linearführung zwischen einer ersten und einer zweiten Position bewegbar, wobei in der ersten Position das Kanülenelement näher zu der zweiten Zugangsöffnung gelegen ist als in der seiner zweiten Position. Die zweite Position ist dabei so gelegen, dass die Kanüle in dieser Position in das Behältnis eindringt, d. h. insbesondere ein Septum des Behältnisses durchsticht. In der Ausgangslage, d. h. im Auslieferungszustand der Füllhilfe, ist das Kanülenelement vorzugsweise in der ersten Position gelegen, in welcher die Kanüle nicht in den Teil des Aufnahmeraumes, in welchem das Behältnis aufgenommen wird, eindringt. Vorzugsweise ist eine durch Krafteinwirkung überwindbare Arretierung bzw. Klemmung für das Kanülenelement in der ersten Position vorgesehen. Bei der Benutzung wird eine Spritze mit einem Luerkonus in die zweite Zugangsöffnung so eingesetzt, dass der Luerkonus mit dem Luer-Anschluss der Kanüle in Eingriff tritt. Bei weiterem Einschieben der Spritze in die zweite Zugangsöffnung wird dann das Kanülenelement aus seiner ersten Position in seine zweite Position bewegt, wobei vorzugsweise eine vorhandene Arretierung oder Klemmung überwunden wird. So wird das Kanülenelement mit der Kanüle in das Behältnis eingeschoben.

Weiter bevorzugt ist in der zweiten Aufnahme zumindest ein Haltemittel angeordnet, welches das Kanülenelement lösbar fixiert, wobei das Haltemittel in Längsrichtung der Kanüle vorzugsweise eine Haltekraft erzeugt, welche geringer ist als eine von dem Luer-Anschluss in Längsrichtung übertragbare Kraft, d. h. übertragbare Zugkraft. Das Haltemittel kann gleichzeitig die Funktion der oben beschriebenen Arretierung bzw. Klemmung übernehmen. Das Haltemittel verhindert, dass das Kanülenelement aus der zweiten Zugangsöffnung herausfallen kann. Bei der Benutzung wird nach dem Transfer der Flüssigkeit aus dem Behältnis in eine eingesetzte Spritze mithilfe des Kanülenelementes das Kanülenelement gemeinsam mit der Spritze aus der zweiten Zugangsöffnung herausgezogen, wobei die Haltekraft des Haltemittels überwunden wird. D. h. dabei muss die Verbindung zwischen Luerkonus und Luer-Anschluss bestehen bleiben, sodass eine Zugkraft zur Überwindung der Haltekraft des Haltemittels übertragen werden kann.

Die zweite Zugangsöffnung ist vorzugsweise so dimensioniert, dass das Kanülenelement durch die zweite Zugangsöffnung entnehmbar ist. Dazu wird das Kanülenelement vorzugsweise gemeinsam mit einer an das Kanülenelement angesetzten Spritze aus der zweiten Zugangsöffnung herausgezogen.

Im nächsten Schritt kann dann vorzugsweise die Spritze mit dem Kanülenelement in die erste Zugangsöffnung eingesetzt werden und die Kanüle dabei in die Öffnung der Zylinderampulle, welche in der ersten Aufnahme gehalten bzw. fixiert ist, eindringen. Dabei durchsticht die Kanüle vorzugsweise einen Verschluss, insbesondere ein Septum der Zylinderampulle. Die erste Zugangsöffnung und die erste Aufnahme sind dabei vorzugsweise so ausgebildet und angeordnet, dass das Kanülenelement in der ersten Aufnahme linear geführt wird, um ein Septum der Zylinderampulle definiert zu durchstechen. Weiter bevorzugt ist eine Führung vorgesehen, welche das Kanülenelement so führt, dass die Kanüle außerhalb der Mitte des Septums in dieses eindringt. Dies hat den Vorteil, dass dort, wo später in einem Pen-System das Septum durchstochen wird, dieses nicht bereits von der Kanüle beim Befüllen durchstochen worden ist, sodass in diesem Bereich eine gute Abdichtung erhalten bleibt.

Gemäß einer bevorzugten Ausführungsform der Erfindung ist Bestandteil der Füllhilfe eine von dem Gehäuse getrennte Spritze mit einem Luerkonus. Diese Spritze ist so ausgebildet, dass der Luerkonus mit dem Luer-Anschluss des zuvor beschriebenen Kanülenelementes in Eingriff bringbar ist. Die Spritze kann entweder als leere Spritze bereitgestellt werden oder kann mit einer Flüssigkeit zum Auflösen eines medizinischen oder pharmazeutischen Stoffes in dem Behältnis vorgefüllt sein, d. h. beispielsweise mit einer Kochsalzlösung oder Wasser für Injektionszwecke. Diese Spritze wird dann in der vorangehend beschriebenen Weise verwendet, um eine medizinische oder pharmazeutische Flüssigkeit aus einem Behältnis in eine Zylinderampulle zu transferieren. Gegebenenfalls kann zunächst die in der Spritze enthaltene Flüssigkeit in das Behältnis gedrückt werden, um dort einen trockenen Stoff aufzulösen und die gewünschte Flüssigkeit zu erzeugen. Nach ausreichender Durchmischung wird dann mit derselben Spritze vorzugsweise die Flüssigkeit aus dem Behältnis herausgesaugt und anschließend die Spritze mit dem Kanülenelement in der vorangehend beschriebenen Weise aus der zweiten Zugangsöffnung entnommen und in die erste Zugangsöffnung eingesetzt, um dann die Flüssigkeit in die in der ersten Aufnahme gehaltene Zylinderampulle zu überführen.

Gemäß einer besonderen Ausführungsform der Erfindung kann in dem Gehäuse eine dritte Aufnahme ausgebildet sein, welche zur Aufnahme und Fixierung einer Spritze ausgebildet ist. Diese dritte Aufnahme ermöglicht es, die Spritze nach der Verwendung in die dritte Aufnahme einzusetzen und dann gemeinsam mit der Füllhilfe zu entsorgen. Vorzugsweise ist die dritte Aufnahme nur zur Aufnahme und Fixierung der Spritze ausgebildet, das Kanülenelement verbleibt besonders bevorzugt, wie nachfolgend beschrieben wird, nach der Benutzung in oder an der ersten Zugangsöffnung.

Vorzugsweise ist die erste Zugangsöffnung derart dimensioniert, dass durch die erste Zugangsöffnung ein Kanülenelement und vorzugsweise das zuvor beschriebene Kanülenelement in die erste Aufnahme einsetzbar ist. Dies ermöglicht es, die Spritze mit dem Kanülenelement, nachdem sie aus der zweiten Zugangsöffnung entnommen worden sind, in die erste Zugangsöffnung einzusetzen, um die Kanüle des Kanülenelementes in die zu befüllende Zylinderampulle einzubringen bzw. einzustechen.

In der ersten Aufnahme ist an der ersten Zugangsöffnung weiter bevorzugt zumindest ein Sicherungsmittel angeordnet, welches so ausgebildet ist, dass es ein durch die erste Zugangsöffnung eingesetztes Kanülenelement fixiert und vorzugsweise nicht wieder entnehmbar fixiert. Dies ermöglicht es, nach dem Einbringen der Flüssigkeit in die Zylinderampulle die Spritze aus der ersten Zugangsöffnung zu entnehmen, während das Kanülenelement dort fixiert bleibt und dort sicher zur Entsorgung verwahrt ist. Das Sicherungsmittel ist somit vorzugsweise so ausgebildet, dass es in einer Richtung beim Einsetzen überwindbar ist und in der entgegengesetzten Bewegungsrichtung sperrt. Dies kann beispielsweise durch elastische Rastzungen bzw. Rastvorsprünge erreicht werden, welche vorzugsweise einstückig mit zumindest einem Gehäuseteil ausgebildet sind. Dies kann beispielsweise das vorangehend beschriebene Gehäuse sein oder aber ein mit dem Gehäuse verbundenes Bauteil, beispielsweise das nachfolgend beschriebene Verschlusselement.

Gemäß der Erfindung weist die Füllhilfe ferner ein zwischen einer ersten und einer zweiten Position bewegliches Verschlusselement auf, welches in der ersten Position die erste Zugangsöffnung verschließt und die zweite Zugangsöffnung freigibt und in seiner zweiten Position die zweite Zugangsöffnung verschließt und die erste Zugangsöffnung freigibt. Das Verschlusselement dient einer sicheren Handhabung, da es in der richtigen Reihenfolge für die Benutzung jeweils nur eine Zugangsöffnung freigibt. D. h. im Auslieferungszustand befindet sich das Verschlusselement vorzugsweise in seiner ersten Position, sodass eine Spritze zunächst in die zweite Zugangsöffnung eingesetzt werden kann. Nachdem die Spritze dann vorzugsweise aus der zweiten Zugangsöffnung entnommen worden ist, kann das Verschlusselement in seine zweite Position bewegt werden, sodass die erste Zugangsöffnung freigegeben wird und die Spritze dann, wiederum bevorzugt mit dem angesetzten Kanülenelement in die erste Zugangsöffnung eingesetzt werden kann. Das Verschlusselement ist vorzugsweise ebenfalls als Spritzgussteil aus Kunststoff ausgebildet und mit dem oben beschriebenen Gehäuse beweglich verbunden. Dies kann beispielsweise durch Verrasten erfolgen. So ist es möglich, das Gehäuse und das Verschlusselement allein durch zwei Bauteile auszubilden, wobei vorzugsweise alle oben beschriebenen Bestandteile des Gehäuses, insbesondere die Haltemittel, Sicherungsmittel, Fixiermittel, Arretierungen und/oder Klemmungen einstückig mit dem Gehäuse ausgebildet sind.

Das vorangehend beschriebene Sicherungsmittel für das Kanülenelement an der ersten Zugangsöffnung kann vorzugsweise durch Rasthaken oder Rastzungen an dem Verschlusselement ausgebildet sein, sodass das Kanülenelement nach dem Einsetzen in die erste Zugangsöffnung zwischen dem Verschlusselement und dem Gehäuse fixiert wird.

Weiter bevorzugt weist das Verschlusselement zumindest eine lösbare Sperre auf, welche das Verschlusselement in seiner ersten und/oder seiner zweiten Position lösbar fixiert, wobei die Sperre vorzugsweise einhändig lösbar ist. Durch eine solche Sperre wird verhindert, dass das Verschlusselement unbeabsichtigt von der ersten in die zweite Position verschoben wird. Eine solche Sperre kann beispielsweise durch einen auf einer federnden Zunge angeordneten Vorsprung an dem Gehäuse gebildet werden, welcher, wenn sich das Verschlusselement in seiner ersten Position befindet, vor einer Kante, insbesondere einer in Verschieberichtung vorderen Kante des Verschlusselementes gelegen ist. So blockiert der Vorsprung die Bewegung des Verschlusselementes. Zur Bewegung des Verschlusselementes muss der Vorsprung dann über die federnde Rastzunge mit der Kante außer Eingriff gebracht werden, sodass das Verschlusselement dann bewegt, insbesondere verschoben werden kann. Die Sperre kann ferner bevorzugt so ausgebildet sein, dass sie dann auch in der zweiten Position des Verschlusselementes mit diesem in geeigneter Weise in Eingriff tritt, um das Verschlusselement in der zweiten Position zu sichern. In dem Verschlusselement kann dazu vorzugsweise eine Ausnehmung oder Öffnung ausgebildet sein, in welche der Vorsprung der federnden Rastzunge eingreift. Eine Anordnung einer solchen Sperre könnte auch in derart umgekehrter Weise erfolgen, dass der Vorsprung in der ersten Position des Verschlusselementes in eine Ausnehmung des Verschlusselementes eingreift und in der zweiten Position des Verschlusselementes an einer in Verschieberichtung hinteren Kante des Verschlusselementes oder einer zweiten Ausnehmung gelegen ist. Besonders bevorzugt sind zwei derartige Sperren vorgesehen, welche an entgegengesetzten Seitenflächen des Gehäuses gelegen sind und mit den Fingern zum Verschieben des Verschlusselementes zusammengedrückt werden müssen.

Weiter bevorzugt ist das Verschlusselement in einer Führung in dem Gehäuse verschiebbar geführt. Dabei ist die Führung vorzugsweise gerade ausgebildet und erstreckt sich quer zu den Einsetzrichtungen der Spritze in die erste und die zweite Zugangsöffnung. Besonders bevorzugt erstrecken sich die Führung und die durch sie definierte Verschieberichtung dabei nicht normal zu der genannten Einsetzrichtung, sondern schräg. Dies bewirkt, dass sich das Verschlusselement beim Verschieben von der ersten in die zweite Position nicht nur quer zu den Einsetzrichtungen, sondern auch ein gewisses Maß in Richtung der Einsetzrichtungen verschiebt. In dem Verschlusselement kann eine Öffnung vorgesehen sein, welche in der zweiten Position die erste Zugangsöffnung überdeckt. Diese Öffnung dient gleichzeitig auch der Führung der Spritze. Wenn das Verschlusselement auch axial in der Einsetzrichtung bewegt wird, wenn es von der ersten in die zweite Position verschoben wird, wird somit die von der Öffnung gebildete Führung in eine andere Axialposition gebracht. Dies ist vorteilhaft, da üblicherweise die zu befüllende Zylinderampulle in axialer Richtung länger als das genannte Behältnis, insbesondere ein Vial, ist. Es ist auf diese Weise möglich, die Füllhilfe besonders kompakt auszubilden, insbesondere in ihrem Auslieferungszustand, in welchem sich das Verschlusselement in der ersten Position befindet, welche zum Einsetzen der Spritze in das Behältnis bestimmt ist. Die zum Führen der Spritze zum Einsetzen in die Zylinderampulle erforderliche axiale Länge der Füllhilfe wird dann erst durch Verschieben des Verschlusselementes in seine zweite Position geschaffen, wodurch sich die Füllhilfe in axialer Richtung verlängert.

Die erste und die zweite Zugangsöffnung sind vorzugsweise an derselben Seite des Gehäuses angeordnet. Dies vereinfacht die Handhabung, da es nicht erforderlich ist, das Gehäuse zu drehen, sondern die Spritze an derselben Seite von der ersten in die zweite Zugangsöffnung umgesetzt werden kann.

Die erste Aufnahme mit der ersten Zugangsöffnung und die zweite Aufnahme mit der zweiten Zugangsöffnung sind weiter bevorzugt derart angeordnet und ausgebildet, dass die Einsetzrichtungen der ersten und der zweiten Zugangsöffnung, entlang derer ein Kanülenelement und/oder eine Spritze in die Zugangsöffnungen einsetzbar bzw. entnehmbar sind, parallel zueinander verlaufen. Auch dies vereinfacht die Handhabung, da die Winkellage des Gehäuses beim Herausziehen und Einsetzen der Spritze nicht geändert werden muss.

Gegenstand der Erfindung ist darüber hinaus ein Verfahren zum Befüllen einer Zylinderampulle mit einer medizinischen oder pharmazeutischen Flüssigkeit unter Verwendung einer Füllhilfe, wie sie vorangehend beschrieben wurde. Die wesentlichen Verfahrensschritte werden nachfolgend beschrieben. Weitere Details des Verfahrensablaufes wurden bereits vorangehend im Zusammenhang mit der Gestaltung der Füllhilfe beschrieben. Auf diese Ausführungen wird hinsichtlich des Verfahrens verwiesen.

Gemäß dem Verfahren wird zunächst ein Behältnis mit der medizinischen oder pharmazeutischen Flüssigkeit bereitgestellt, welche in die Zylinderampulle transferiert werden soll. Dieses Behältnis wird in die zweite Aufnahme des Gehäuses der Füllhilfe eingesetzt oder ist im Auslieferungszustand dort bereits eingesetzt. Im nächsten Schritt wird eine Spritze in die zweite Zugangsöffnung an der zweiten Aufnahme in dem Gehäuse eingesetzt und mit dem Inneren des Behältnisses in fluidleitende Verbindung gebracht. Dies erfolgt bevorzugt in der Weise, dass, wie oben beschrieben, eine Kanüle eines Kanülenelementes, welches bereits vormontiert in der zweiten Aufnahme gehalten ist, in die Öffnung des Behältnisses eingestochen wird. Im nächsten Schritt wird die medizinische oder pharmazeutische Flüssigkeit aus dem Behältnis in die Spritze transferiert. Dies kann insbesondere durch Ansaugen mit Hilfe der Spritze geschehen. Alternativ kann jedoch auch ein Behältnis mit einem Kolben vorgesehen sein, sodass der Transfer durch Druck auf den Kolben in dem Behältnis erfolgen kann. Nach dem Transfer der Flüssigkeit in die Spritze wird die Spritze aus der zweiten Zugangsöffnung entnommen, wobei vorzugsweise ein an der Spritze angeordnetes Kanülenelement mit entnommen wird. Anschließend wird die Spritze, gegebenenfalls nach vorherigem Verschieben eines Verschlusselementes, in die erste Zugangsöffnung eingesetzt. Dabei wird, sofern vorhanden, die beschriebene Kanüle in die Öffnung der Zylinderampulle eingestochen. Anschließend wird die medizinische oder pharmazeutische Flüssigkeit aus der Spritze in eine in der ersten Aufnahme angeordnete Zylinderampulle gedrückt. Die Zylinderampulle ist vorzugsweise bereits vormontiert in der ersten Aufnahme gehalten, sodass die Füllhilfe mit montierter Zylinderampulle ausgeliefert wird. Nachdem die Flüssigkeit aus der Spritze in die Zylinderampulle überführt worden ist, kann die Zylinderampulle dann aus der ersten Aufnahme entnommen werden und in bekannter Weise in eine Kapulen-Spritze oder ein Pen-System eingesetzt werden. Die Spritze kann dann, wie oben beschrieben, aus der ersten Zugangsöffnung wieder entnommen werden, wobei vorzugsweise ein Kanülenelement in der Aufnahme verbleibt. Gegebenenfalls kann dann die Spritze in eine dritte Aufnahme, wie es oben beschrieben wurde, eingesetzt werden.

Gemäß einer besonderen Ausführungsform des Verfahrens kann in dem Behältnis zunächst ein pulverförmiger, beispielsweise gefriergetrockneter medizinischer oder pharmazeutischer Stoff enthalten sein, welcher unter Zusatz einer Flüssigkeit aufgelöst wird, um so die genannte medizinische oder pharmazeutische Flüssigkeit in dem Behältnis zu erzeugen. Dazu wird vorzugsweise mit der Spritze ein Lösungsmittel, beispielsweise Kochsalzlösung oder Wasser für Injektionszwecke, wenn die Spritze in die zweite Zugangsöffnung eingesetzt worden ist, in das Behältnis gedrückt, woraufhin der Stoff in dem Behältnis durch die Flüssigkeit aufgelöst wird. Dies kann gegebenenfalls unter Schütteln erfolgen. Nach erfolgter Durchmischung bzw. erfolgtem Auflösen wird dann die so erzeugte medizinische oder pharmazeutische Flüssigkeit vorzugsweise wieder in dieselbe Spritze transferiert, wie es oben beschrieben wurde. Dann wird die Spritze in der beschriebenen Weise aus der zweiten Zugangsöffnung entnommen und in die erste Zugangsöffnung eingesetzt.

Ein erfindungsgemäßer Vorteil des Verfahrens liegt darin, dass die medizinische oder pharmazeutische Flüssigkeit aus der Spritze in die Zylinderampulle gedrückt wird und nicht durch Bewegung des beweglichen Stopfens in der Zylinderampulle angesaugt wird, wie es bei herkömmlicher Befüllung der Zylinderampullen üblich ist. Dies ist von Vorteil, da so ein unerwünschtes Aufschäumen der Flüssigkeit verhindert werden kann. Um dies auch beim Transfer von dem Behältnis in die Spritze zu verhindern, kann das Behältnis auch so ausgebildet sein, dass es einen beweglichen Kolben aufweist, welcher gedrückt wird, um die Flüssigkeit aus dem Behältnis in eine angesetzte Spritze zu drücken.

Nachfolgend wird die Erfindung beispielhaft anhand der beigefügten Figuren beschrieben. In diesen zeigt:
- Fig. 1: die wesentlichen Teile einer erfindungsgemäßen Füllhilfe mit einem Behältnis für eine medizinische oder pharmazeutische Flüssigkeit sowie eine Zylinderampulle,
- Fig. 2: eine perspektivische Ansicht des Gehäuses der Füllhilfe mit angesetztem Verschlusselement,
- Fig. 3: eine Seitenansicht des Gehäuses gemäß Fig. 2,
- Fig. 4: eine Draufsicht auf das Gehäuse gemäß Fig. 3,
- Fig. 5: eine Seitenansicht des Gehäuses ohne Verschlusselement,
- Fig. 6: eine Seitenansicht des Gehäuses gemäß Fig. 5 mit Blick auf eine der Stirnseiten,
- Fig. 7: eine Schnittansicht durch das Gehäuse gemäß Fig. 5 entlang der Linie VII in Fig. 6,
- Fig. 8: eine perspektivische Ansicht des Verschlusselementes,
- Fig. 9: eine perspektivische Ansicht der Füllhilfe mit eingesetzter Zylinderampulle und eingesetztem Behältnis,
- Fig. 10 - 20: schrittweise die Verwendung der erfindungsgemäßen Füllhilfe und

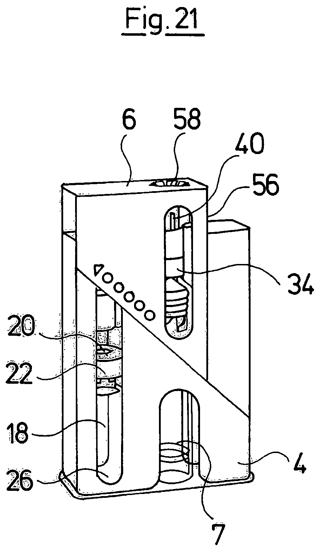

- Fig. 21: eine perspektivische Ansicht der Füllhilfe nach Befüllen der Zylinderampulle.

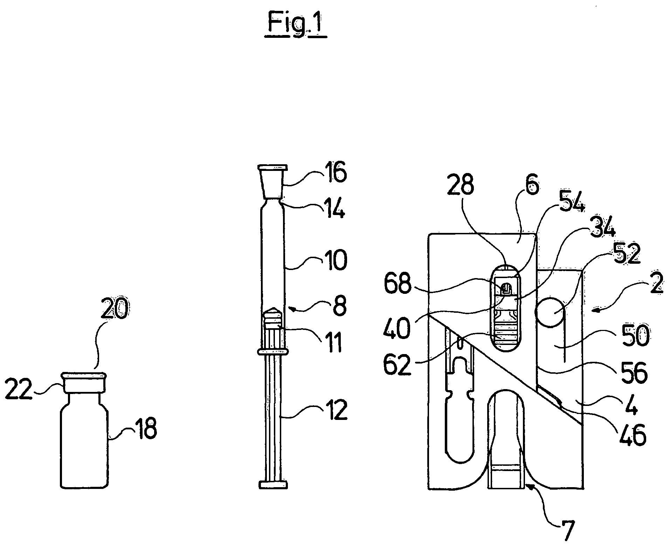

Die hier als Beispiel gezeigte Füllhilfe weist zwei wesentliche Komponenten auf, nämlich zum einen eine Haltevorrichtung 2, welche aus einem Gehäuse 4 mit einem daran angeordneten Verschlusselement 6 gebildet ist, und zum anderen eine Spritze 8. Die Haltevorrichtung 2 dient zur Aufnahme einer zu befüllenden Zylinderampulle 7. Die Spritze 8 ist in üblicher Weise aus einem Zylinder 10 mit einem darin über eine Kolbenstange 12 beweglichen Kolben 11 ausgebildet. Die Spritze 8 weist an ihrem Ende einen Luerkonus 14 auf, auf welchem in den in Fig. 1 gezeigten Beispiel eine Kappe 16 aufgesetzt ist.

Die erfindungsgemäße Füllhilfe dient dazu, eine medizinische oder pharmazeutische Flüssigkeit aus einem Behältnis 18 in die Zylinderampulle 7 zu überführen. Bei dem hier gezeigten Behältnis 18 handelt es sich um ein übliches Vial bzw. Injektionsfläschchen, welches an seiner Oberseite durch einen Stopfen bzw. ein Septum 20 verschlossen ist. Das Septum 20 umgebend ist an dem Behältnis 18 ein Kragen 22 ausgebildet. Das Gehäuse 4 weist eine erste Aufnahme 24 und eine zweite Aufnahme 26 auf, wobei die erste Aufnahme zur Aufnahme der Zylinderampulle 7 und die zweite Aufnahme 26 zur Aufnahme des Behältnisses 18 ausgebildet, d. h. entsprechend geformt und dimensioniert ist. Die erste Aufnahme 24 und die zweite Aufnahme 26 sind zu derselben Seite des Gehäuses 4 hin geöffnet und weisen zueinander parallele Einsetzrichtungen E1 und E2 auf, entlang derer die Zylinderampulle 7 und das Behältnis 18 in die erste Aufnahme 24 bzw. die zweite Aufnahme 26 eingesetzt werden. An der ersten Aufnahme 24 ist eine erste Zugangsöffnung 28 angeordnet, welche sich zu einer in der Einsetzrichtung E1 entgegengesetzten Stirnseite des Gehäuses 4 öffnet. Die zweite Aufnahme 26 weist entsprechend eine zweite Zugangsöffnung 30 auf, welche sich zu derselben Seite wie die erste Zugangsöffnung 28 öffnet.

In der ersten Aufnahme 24 sind nahe der ersten Zugangsöffnung 28 einstückig mit dem Gehäuse 4 ausgebildete federnde Rastzungen 32 angeordnet. Diese Rastzungen 32 sind so angeordnet und ausgebildet, dass sie einen Kragen 34, welcher einen Stopfen- bzw. Septum der Zylinderampulle 7 umgibt, umgreifen können, um die Zylinderampulle 7 lösbar in der Aufnahme 24 zu fixieren. Wenn die Zylinderampulle 7 in dieser Weise in der Aufnahme 24 fixiert ist, liegt die von dem Septum verschlossene Öffnung der Zylinderampulle 7 der ersten Zugangsöffnung 28 gegenüber.

Die Rastzungen 32, welche Fixiermittel bilden, sind so angeordnet und ausgebildet, dass sie sich bei ausreichend großer Krafteinwirkung in der Einsetzrichtung E1 von der ersten Zugangsöffnung 28 weg radial nach außen verformen, sodass der Kragen 34 die Rastzungen 32 passieren kann und die Zylinderampulle aus der ersten Aufnahme 24 entnommen werden kann.

In der zweiten Aufnahme 26 sind Fixiermittel in Form von Rastzungen 36 angeordnet, welche so dimensioniert und angeordnet sind, dass sie den Kragen 22 des Behältnisses 18 hintergreifen können, um das Behältnis 18 in der zweiten Aufnahme 26 zu fixieren. Dabei können die Rastzungen 36 mit ihren Vorsprüngen so ausgebildet sein, dass das Behältnis 18 nicht wieder aus der zweiten Aufnahme 26 entnehmbar ist, d. h. die Rastzungen 36 weiten sich nur einmalig beim Einsetzen des Behältnisses in die zweite Aufnahme 26 auf und sind nicht dafür vorgesehen, das Behältnis 18 wieder freizugeben. Alternativ können die Rastzungen 36 jedoch auch entsprechend der Rastzungen 32 so ausgebildet sein, dass es möglich ist, das Behältnis 18 wieder zu entnehmen.

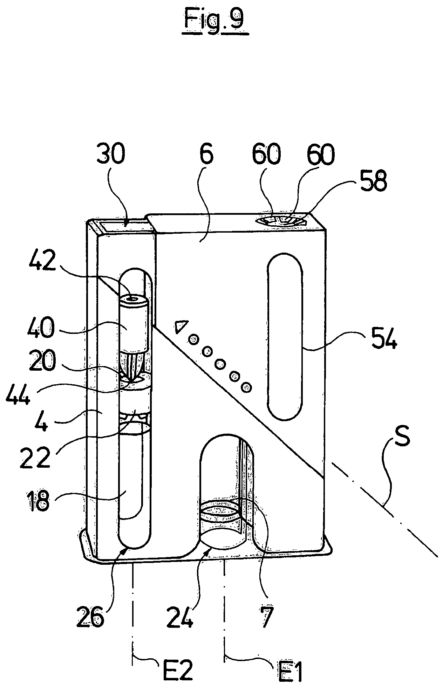

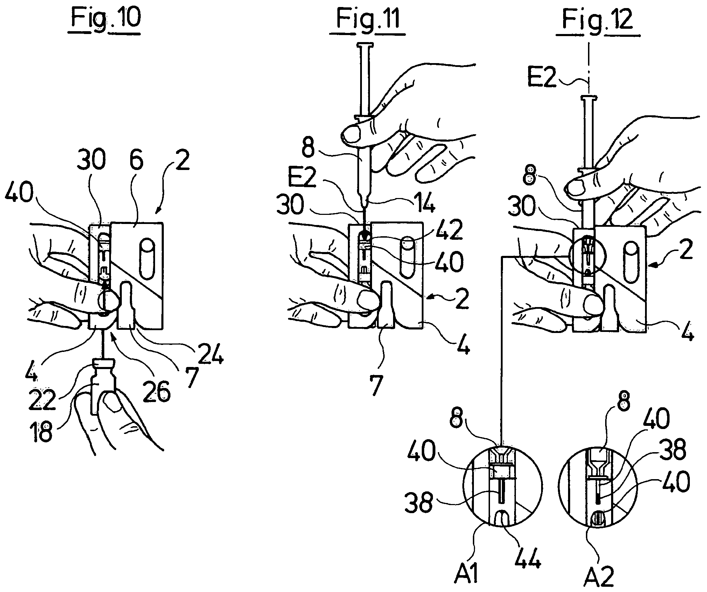

In der zweiten Aufnahme 26 ist zwischen den Rastzungen 36 und der zweiten Zugangsöffnung 30 ein Haltemittel 38 in Form einer Klemmung ausgebildet. Das Haltemittel 38 dient zur Aufnahme und Fixierung eines Kanülenelementes 40. Dabei wird das Kanülenelement 40 von den Haltemitteln 38 axial verschiebbar in Richtung der Einsetzrichtung E2 geführt. Das Kanülenelement 40 weist an seinem der zweiten Zugangsöffnung 30 zugewandten Ende einen Luer-Anschluss 42 und an seinem entgegengesetzten Ende eine Kanüle 44 auf. Das Kanülenelement 40 ist in Richtung der Einsetzrichtung E2 zwischen einer ersten Position, welche in dem Ausschnitt A1 in Fig. 12 gezeigt ist, und einer zweiten Position, welche in dem Ausschnitt A2 in Fig. 12 gezeigt ist, verschiebbar. In der zweiten Position dringt die Kanüle 44 in das Septum 20 des Behältnisses 18 ein, wie beispielsweise in Fig. 9 gezeigt ist. Die zweite Zugangsöffnung 30 ist so dimensioniert, dass das Kanülenelement 40 aus der zweiten Zugangsöffnung 30 entnehmbar ist.

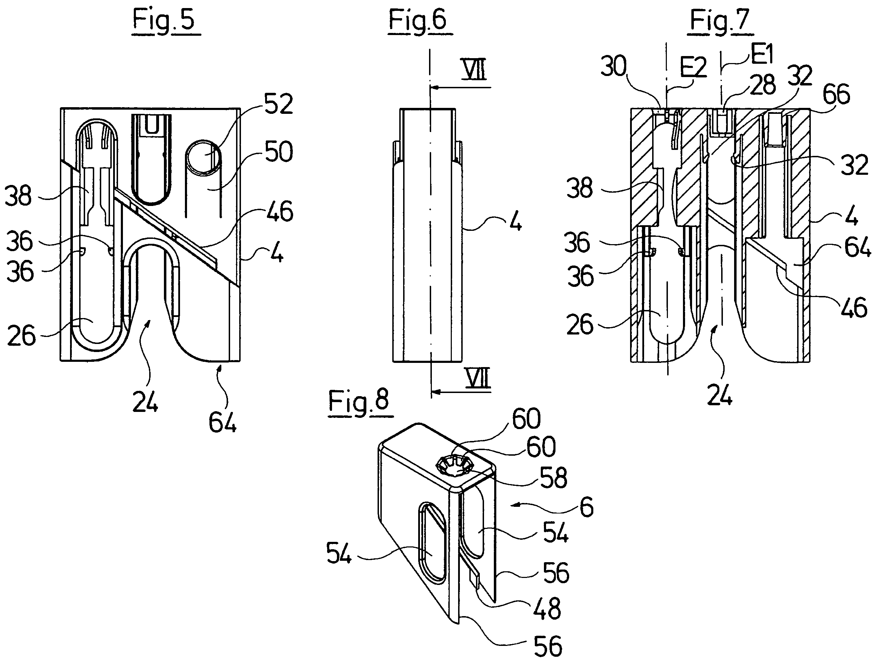

Das Verschlusselement 6 ist als ein auf dem Gehäuse 4 verschiebbarer Schieber ausgebildet, welcher das Gehäuse 4 an zwei Seiten übergreift. D. h. das Verschlusselement 6 weist einen U-förmigen Querschnitt auf und übergreift das Gehäuse 4 an zwei einander abgewandten Seitenflächen. Die Bewegungs- bzw. Verschieberichtung S des Verschlusselementes 6 erstreckt sich quer zu den Einsetzrichtungen E1 und E2. Dabei verläuft die Verschieberichtung S nicht normal, sondern schräg zu den Einsetzrichtungen E1 und E2. Das Gehäuse 4 weist dazu an seinen zwei einander abgewandten Seitenflächen jeweils eine Führungsnut 46 auf, welche sich in der Verschieberichtung S erstrecken. Das Verschlusselement 6 weist an seinen zwei einander zugewandten Innenflächen jeweils einen Vorsprung 48 (in Fig. 8 nur einer sichtbar) auf, welche in die Führungsnuten 46 eingreifen.

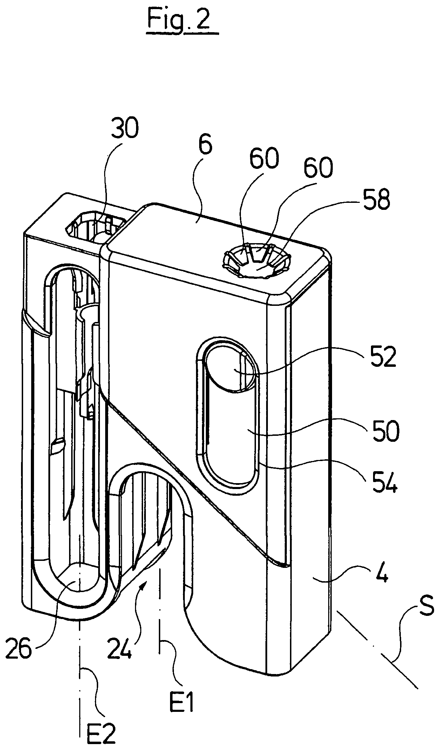

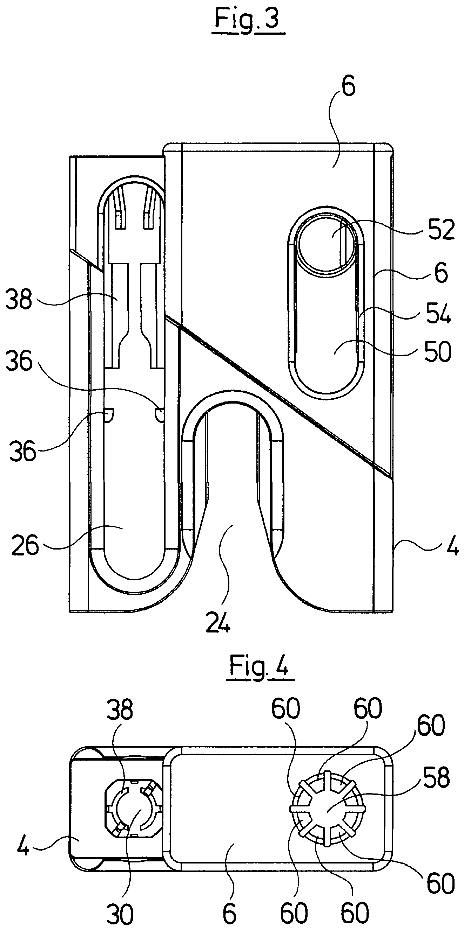

Das Verschlusselement 6 ist so ausgebildet, dass es in einer ersten in den Figuren 2 bis 4 und 9 gezeigten Stellung die erste Zugangsöffnung 28 verschließt und die zweite Zugangsöffnung 30 freigibt. In dieser Position ist das Verschlusselement 6 durch eine Sperre gegen unbeabsichtigtes Verschieben gesichert. Diese Sperre wird von zwei federnden Zungen 50 an zwei voneinander abgewandten Seitenflächen des Gehäuses 4 gebildet. Die Zungen 50 weisen jeweils nach außen auskragende Vorsprünge 52 an ihren freien Enden auf. In der ersten Stellung des Schiebers greifen diese Vorsprünge 52 jeweils in eine Öffnung 54 an dem Verschlusselement 6 ein. Um das Verschlusselement 6 in seine zweite Position verschieben zu können, ist es erforderlich, von außen die zwei Vorsprünge 52 zusammenzudrücken, sodass sie von den Öffnungen 54 außer Eingriff treten. In der zweiten Position federn die Zungen 50 dann wieder nach außen zurück und die Vorsprünge 52 kommen an einer Seitenkante 56 zu liegen, wie beispielsweise in Fig. 1 gezeigt ist. So ist das Verschlusselement 6 in seiner zweiten Position gesichert. In dieser zweiten Position liegt eine Öffnung 58, welche an der Stirnseite des Verschlusselementes 6 ausgebildet ist, der ersten Zugangsöffnung 28 gegenüber. Die Öffnung 58 weist in ihrem Umfang Federzungen 60 auf, welche ein Sicherungsmittel zum Halten des Kanülenelements 40 bilden. Die Öffnung 58 und die Federzungen 60 sind so dimensioniert, dass das Kanülenelement 40 die Öffnung 58 unter radialer Aufweitung der Federzungen 60 passieren kann. Wenn das Kanülenelement 40 die Öffnung 58 passiert hat, federn die Federzungen 60 wieder radial nach innen und verhindern eine Rückbewegung des Kanülenelementes 40.

Die Funktionsweise der erfindungsgemäßen Füllhilfe wird nun anhand der Figuren 10 bis 20 erläutert. In diesem Ausführungsbeispiel ist das Behältnis 18 mit einem pulverförmigen medizinischen oder pharmazeutischen Stoff vorgefüllt. Die Haltevorrichtung 2 wird, wie in Fig. 10 gezeigt ist, in einem Zustand ausgeliefert, in welchem sich das Verschlusselement in seiner ersten Position befindet. Ferner ist die Zylinderampulle 7 in dem Gehäuse 4 in der ersten Aufnahme 24 vormontiert. Sie wird dort in der oben beschriebenen Weise durch die Rastzungen 32 gehalten. In diesem Zustand befindet sich ein beweglicher Kolben 62 in der in Fig. 1 gezeigten Position, d. h. nahe der Öffnung bzw. dem Septum der Zylinderampulle 7, welches von dem Kragen 34 gehalten wird. Ferner ist in dem Gehäuse 4 das Kanülenelement 40 vormontiert, wobei es sich zunächst in seiner ersten, in dem Ausschnitt A1 in Fig. 12 gezeigten Position, befindet, d. h. in einer der zweiten Zugangsöffnung 30 zugewandten Position. In diesem Zustand wird das Behältnis 18 in der zweiten Einsetzrichtung E2 in die zweite Aufnahme 26 eingeschoben, wobei sich die Rastzungen 36 radial aufweiten, sodass der Kragen 22 die Vorsprünge der Rastzungen 36 passieren kann und anschließend von den Vorsprüngen der Rastzungen 36 umgriffen wird, sodass das Behältnis 18 in der zweiten Aufnahme 26 fixiert ist.

Im nächsten Schritt wird nach Abnehmen der Kappe 16 die Spritze 8, wie in Fig. 11 gezeigt, in die zweite Zugangsöffnung 30 eingesetzt. Dabei tritt der Luerkonus 14 der Sprite 8 mit dem Luer-Anschluss 42 des Kanülenelementes 40 in Eingriff. In diesem Beispiel ist die Spritze 8 mit einer Flüssigkeit zum Auflösen des medizinischen oder pharmazeutischen Stoffes, beispielsweise einer Kochsalzlösung oder Wasser für Injektionszwecke, vorgefüllt.

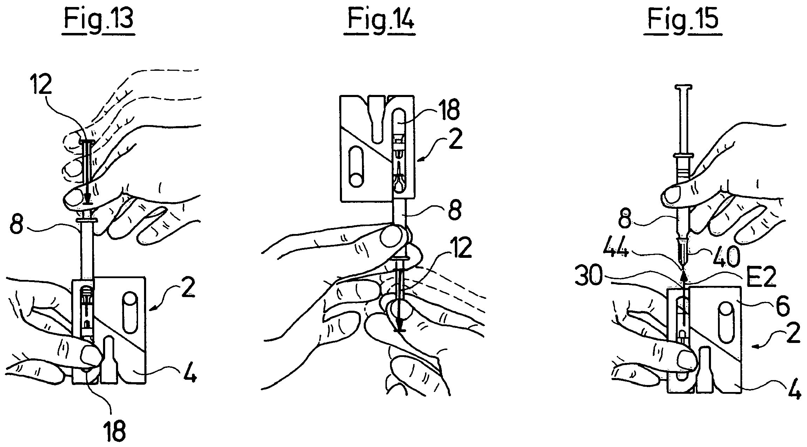

Wenn die Spritze 8 mit ihrem Luerkonus 14 mit dem Kanülenelement 40 in Eingriff getreten ist, wie in Fig. 12 gezeigt, wird die Spritze 8 in der Einsetzrichtung E2 weiter vorgeschoben, sodass, wie im Ausschnitt A2 in Fig. 12 gezeigt, das Kanülenelement 40 in seine zweite, weiter von der zweiten Zugangsöffnung 30 beabstandete Position bewegt wird. In dieser Position sticht die Kanüle 44 in das Septum 20 des Behältnisses 18 ein. Im nächsten Schritt wird, wie in Fig. 13 gezeigt, die Kolbenstange 12 der Spritze 8 verschoben und so der Inhalt der Spritze 8 durch das Kanülenelement 40 in das Behältnis 18 hinein entleert. So wird der Stoff in dem Behältnis 18 aufgelöst, wobei hierzu gegebenenfalls die Haltevorrichtung 2, d. h. das Gehäuse 4, geschüttelt werden kann. Im nächsten Schritt wird, wie in Fig. 14 gezeigt, die Haltevorrichtung 2 mit der eingesetzten Spritze 8 umgedreht, sodass die Kolbenstange 12 nach unten gerichtet ist. In diesem Zustand wird die Spritze 8 aufgezogen, sodass der Inhalt des Behältnisses 18, welcher nun eine medizinische oder pharmazeutische Flüssigkeit ist, in die Spritze 8 aufgezogen wird.

Im nächsten Schritt wird, wie in Fig. 15 gezeigt, die Haltevorrichtung 2 wieder umgedreht und die Spritze 8 wird in der Einsetzrichtung E2 gemeinsam mit dem Kanülenelement 40 aus der zweiten Zugangsöffnung herausgezogen, wobei die Kanüle 44 aus dem Septum 20 herausgezogen wird. Dazu ist es erforderlich, dass die Haltekraft in den Haltemitteln 38 geringer ist als die Klemmkraft zwischen Luerkonus 14 und Luer-Anschluss 42.

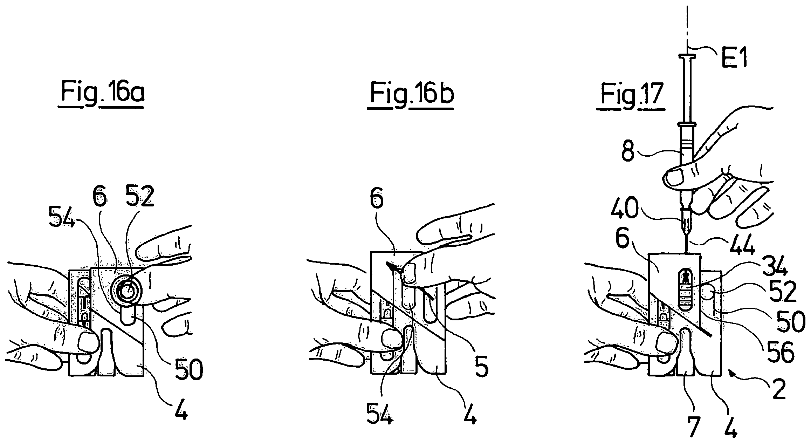

Im nächsten Schritt wird, wie in Fig. 16a und Fig. 16b gezeigt, das Verschlusselement 6 entlang der Verschieberichtung S an dem Gehäuse 4 verschoben. Dazu werden zunächst, wie in Fig. 16a gezeigt, die Vorsprünge 52 (in Fig. 16a ist nur der an der Vorderseite gelegene Vorsprung sichtbar, an der Rückseite ist ein zweiter, identisch ausgebildeter Vorsprung 52 gelegen) zusammengedrückt, sodass die Vorsprünge 52 von den Öffnungen 54 außer Eingriff treten. Anschließend kann, wie in Fig. 16 b gezeigt, das Verschlusselement 6 in der Verschieberichtung S verschoben werden. Dabei ist eine Einhandbedienung möglich. D. h. das Zusammendrücken der Vorsprünge 52 und das Verschieben des Verschlusselementes 6 kann ohne Umgreifen mit einer Hand geschehen, da zum Verschieben des Verschlusselementes 6 dieses im Bereich der Öffnungen 54 gehalten werden kann. So gelangt das Verschlusselement 6 in seine in Fig. 17 gezeigte zweite Position. In dieser Position federn die Vorsprünge 52 an den Zungen 50 wieder nach außen, sodass sie an den Seitenkanten 56 des Verschlusselementes 6 zur Anlage kommen und dieses in der gezeigten zweiten Position sichern.

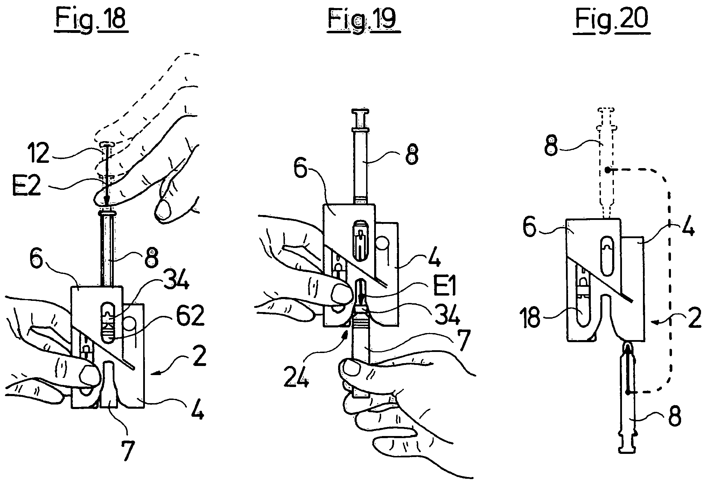

In dieser Position wird die Spritze 8 mit dem Kanülenelement 40 mit der Kanüle 44 voran in der Einsetzrichtung E1 zunächst in die Öffnung 58 an dem Verschlusselement 6 und nachfolgend in die nun darunter liegende erste Zugangsöffnung 28 des Gehäuses 4 eingeführt. Dabei passiert das Kanülenelement 40 in der zuvor beschriebenen Weise die Federzungen 60 an der Öffnung 58. Gleichzeitig tritt die Kanüle 44 durch den Verschluss bzw. das Septum in die Zylinderampulle 7 ein. In diesem Zustand wird nun, wie in Fig. 18 gezeigt, die Kolbenstange 12 der Spritze 8 in der Einsetzrichtung E2 verschoben und es wird der Inhalt der Spritze 8 in die Zylinderampulle 7 gedrückt bzw. überführt. Dabei verschiebt sich der Kolben 62 in der Zylinderampulle 7 zu derem ihrer Öffnung abgewandten Ende. Im nächsten Schritt wird, wie in Fig. 19 gezeigt, die Zylinderampulle 7 in der Einsetzrichtung E1 aus der ersten Aufnahme 24 herausgezogen. Dabei weiten sich die Rastzungen 32 radial auf, sodass der Kragen 34 der Zylinderampulle 7 die Vorsprünge der Rastzungen 32 passieren kann. Die Spritze 8 verbleibt dabei zunächst in der ersten Zugangsöffnung 28.

Die erste Zugangsöffnung 28 weist eine Führung 68 für das Kanülenelement 40 auf, welche das Kanülenelement 40 so führt, dass die Kanüle 44 an definierter Position in den Verschluss bzw. ein Septum der Zylinderampulle 7 eintritt. Dabei ist die Führung vorzugsweise so gewählt, dass die Kanüle 44 außenmittig in das Septum der Zylinderampulle 7 eintritt. D. h. die Führung 68 ist mit ihrer Längs- bzw. Mittelachse radial geringfügig gegenüber der Mittelachse der ersten Aufnahme 24 versetzt. Dies bewirkt, dass das Septum der Zylinderampulle 7 an einer anderen Stelle durchstochen wird, als es später in einem Pen-System bei der Verwendung der Zylinderampulle 7 der Fall sein wird.

Im Anschluss kann, wie in Fig. 20 gezeigt, die Spritze 8 aus der Öffnung 58 wieder entnommen werden, wobei sich dabei der Luerkonus 14 der Spritze 8 wieder von dem Luer-Anschluss 42 des Kanülenelementes 40 löst, da das Kanülenelement 40, wie oben beschrieben, von den Federzungen 60 zurückgehalten wird. Anschließend kann die Spritze 8 in eine dritte Aufnahme 64 (siehe Fig. 7) in dem Gehäuse 4 eingesetzt werden. In der dritten Aufnahme 64 ist ein Klemmelement 66 ausgebildet, welches in seiner Form an den Luerkonus 14 derart angepasst ist, dass dieser in dem Klemmelement 66 geklemmt wird. In diesem Zustand ist dann die Spritze 8 in dem Gehäuse 4 fixiert. Gleichzeitig ist das Kanülenelement 40 zwischen dem Verschlusselement 6 und dem Gehäuse 4 in der beschriebenen Weise fixiert und das entleerte Behältnis 18 weiter in der zweiten Aufnahme 26 fixiert. In diesem Zustand kann die Haltevorrichtung 2 dann gemeinsam mit dem Kanülenelement 40 der Spritze 8 und dem Behältnis 18 entsorgt werden.

### Bezugszeichen

- 2: Haltevorrichtung
- 4: Gehäuse
- 6: Verschlusselement
- 7: Zylinderampulle
- 8: Spritze
- 10: Zylinder
- 11: Kolben
- 12: Kolbenstange
- 14: Luerkonus
- 16: Kappe
- 18: Behältnis
- 20: Septum
- 22: Kragen
- 24: erste Aufnahme
- 26: zweite Aufnahme
- 28: erste Zugangsöffnung
- 30: zweite Zugangsöffnung
- 32: Rastzungen
- 34: Kragen
- 36: Rastzungen
- 38: Haltemittel
- 40: Kanülenelement
- 42: Luer-Anschluss
- 44: Kanüle
- 46: Führungsnuten
- 48: Vorsprung
- 50: Zungen
- 52: Vorsprünge
- 54: Öffnungen
- 56: Seitenkanten
- 58: Öffnung
- 60: Federzungen
- 62: Kolben
- 64: dritte Aufnahme
- 66: Klemmelement

- E1, E2: Einsetzrichtungen
- S: Verschieberichtung

## Patentansprüche

1. Füllhilfe zum Befüllen einer Zylinderampulle (7) mit einer medizinischen oder pharmazeutischen Flüssigkeit, wobei
die Füllhilfe in einem gemeinsamen Gehäuse (4) eine erste Aufnahme (24), welche zur Aufnahme der zu befüllenden Zylinderampulle (7) ausgebildet ist, und eine zweite Aufnahme (26), welche zur Aufnahme eines einen medizinischen oder pharmazeutischen Stoff enthaltenden Behältnisses (18) ausgebildet ist, aufweist,
in der ersten Aufnahme (24) Fixiermittel (32) angeordnet sind, welche dazu ausgebildet und vorgesehen sind, eine Zylinderampulle (7) in der ersten Aufnahme (24) lösbar zu fixieren,
in der zweiten Aufnahme (26) Fixiermittel (36) angeordnet sind, welche dazu ausgebildet und vorgesehen sind, ein einen medizinischen oder pharmazeutischen Stoff enthaltendes Behältnis (18) in der zweiten Aufnahme (26) zu fixieren,
an der ersten Aufnahme (24) eine erste Zugangsöffnung (28) ausgebildet ist, welche derart angeordnet ist, dass sie einer Öffnung einer Zylinderampulle (7), wenn sie in die erste Aufnahme (24) eingesetzt ist, gegenüberliegt und dass die Öffnung der Zylinderampulle (7) durch die erste Zugangsöffnung (28) von außen zugänflich ist, und
das Gehäuse (4) eine zweite Zugangsöffnung (30) aufweist, welche an der zweiten Aufnahme (26) ausgebildet ist und derart angeordnet ist, dass sie einer Öffnung eines Behältnisses (18), wenn es in die zweite Aufnahme (26) eingesetzt ist, gegenüberliegt und dass eine Öffnung des Behältnisses durch die zweite Zugangsöffnung (30) zugänglich ist, **dadurch gekennzeichnet, dass**
die Füllhilfe ein zwischen einer ersten und einer zweiten Position bewegliches Verschlusselement (6) aufweist, welches in der ersten Position die erste Zugangsöffnung (28) verschließt und die zweite Zugangsöffnung (30) freigibt und in seiner zweiten Position die zweite Zugangsöffnung (30) verschließt und die erste Zugangsöffnung (28) freigibt.

2. Füllhilfe nach Anspruch 1, bei welcher eine Zylinderampulle (7) in der ersten Aufnahme (24) mit den Fixiermitteln (32) vormontiert und lösbar fixiert ist sowie aus der ersten Aufnahme (24) entnehmbar ist.

3. Füllhilfe nach Anspruch 1 oder 2, bei welcher die in der zweiten Aufnahme (26) angeordneten Fixiermittel (36) dazu ausgebildet und vorgesehen sind, ein einen medizinischen oder pharmazeutischen Stoff enthaltendes Behältnis (18) in Form eines Vials in der zweiten Aufnahme (26) lösbar zu fixieren.

4. Füllhilfe nach einem der vorangehenden Ansprüche, bei welcher in der zweiten Aufnahme (26) ein Kanülenelement (40), welches eine Kanüle (44) und einen Luer-Anschluss (42) aufweist, lösbar gehalten ist, wobei der Luer-Anschluss (42) des Kanülenelements (40) der zweiten Zugangsöffnung (30) zugewandt ist und die Kanüle (44) so gerichtet ist, dass sie in ein in die zweite Aufnahme (26) eingesetztes Behältnis (18) eindringt.

5. Füllhilfe nach Anspruch 4, bei welcher die zweite Zugangsöffnung (30) so dimensioniert ist, dass das Kanülenelement (40) durch die zweite Zugangsöffnung (30) entnehmbar ist.

6. Füllhilfe nach einem der vorangehenden Ansprüche, bei welcher in der zweiten Aufnahme zumindest ein Haltemittel (38) angeordnet ist, welches das Kanülenelement (40) lösbar fixiert, wobei das Haltemittel (38) in Längsrichtung der Kanüle (44) vorzugsweise eine Haltekraft erzeugt, welche geringer ist als eine von dem Luer-Anschluss (42) in Längsrichtung übertragbare Kraft.

7. Füllhilfe nach einem der vorangehenden Ansprüche, welche eine von dem Gehäuse (4) getrennte Spritze mit einem Luerkonus (14) aufweist, welcher mit dem Luer-Anschluss (42) des Kanülenelementes (40) in Eingriff bringbar ist.

8. Füllhilfe nach einem der vorangehenden Ansprüche, bei welcher das Gehäuse (4) eine dritte Aufnahme (64) aufweist, welche zur Aufnahme und Fixierung einer Spritze (8) ausgebildet ist.

9. Füllhilfe nach einem der vorangehenden Ansprüche, bei welcher die erste Zugangsöffnung (28) derart dimensioniert ist, dass durch die erste Zugangsöffnung (28) ein Kanülenelement (40) und vorzugsweise das in den Ansprüchen 4 bis 8 definierte Kanülenelement (40) in die erste Aufnahme (24) einsetzbar ist.

10. Füllhilfe nach Anspruch 9, bei welcher in der ersten Aufnahme (24) an der ersten Zugangsöffnung (28) zumindest ein Sicherungsmittel (60) angeordnet ist, welches so ausgebildet ist, dass es ein durch die erste Zugangsöffnung (28) eingesetztes Kanülenelement (40) fixiert und vorzugsweise nicht wieder entnehmbar fixiert.

11. Füllhilfe nach Anspruch 10, bei welcher das zumindest eine Sicherungsmittel als Rastelement und insbesondere als Rastzunge (60) ausgebildet ist.

12. Füllhilfe nach einem der vorhergehenden Ansprüche, bei welcher das Verschlusselement (6) zumindest eine lösbare Sperre (50, 52) aufweist, welche das Verschlusselement (6) in seiner ersten und/oder seiner zweiten Position lösbar fixiert, wobei die Sperre (50, 52) vorzugsweise einhändig lösbar ist.

13. Füllhilfe nach einem der vorangehenden Ansprüche, bei welcher die erste (28) und die zweite (30) Zugangsöffnung an derselben Seite des Gehäuses (4) angeordnet sind.

14. Füllhilfe nach einem der vorangehenden Ansprüche, dass die erste Aufnahme (24) mit der ersten Zugangsöffnung (28) und die zweite Aufnahme (26) mit der zweiten Zugangsöffnung (30) derart ausgebildet sind, dass die Einsetzrichtungen (E1, E2) der ersten und der zweiten Zugangsöffnung (28, 30), entlang derer ein Kanülenelement (40) und/oder eine Spritze (8) in die Zugangsöffnungen (28, 30) einsetzbar ist, parallel zueinander verlaufen.

15. Verfahren zum Befüllen einer Zylinderampulle (7) mit einer medizinischen oder pharmazeutischen Flüssigkeit unter Verwendung einer Füllhilfe nach einem der vorangehenden Ansprüche, bei welchem nacheinander ein Behältnis (18) mit der in die Zylinderampulle (7) einzubringenden medizinischen oder pharmazeutischen Flüssigkeit in die zweite Aufnahme (26) eingesetzt ist oder eingesetzt wird,
eine Spritze (8) in die zweite Zugangsöffnung (30) eingesetzt wird, die medizinische oder pharmazeutische Flüssigkeit aus dem Behältnis (18) in die Spritze (8) gesaugt wird,
die Spritze (8) aus der zweiten Zugangsöffnung (30) entnommen und in die erste Zugangsöffnung (28) eingesetzt wird und
die medizinischen oder pharmazeutische Flüssigkeit aus der Spritze (8) in eine in der ersten Aufnahme (24) angeordnete Zylinderampulle (7) überführt wird.

16. Verfahren nach Anspruch 15, bei welchem in dem Behältnis (18) zunächst ein pulverförmiger medizinischer oder pharmazeutischer Stoff enthalten ist, welcher unter Zusatz einer Flüssigkeit aufgelöst wird, um so die medizinische oder pharmazeutische Flüssigkeit in dem Behältnis (18) zu erzeugen.

17. Verfahren nach Anspruch 16, bei welchem in dem Behältnis (18) zunächst ein pulverförmiger medizinischer oder pharmazeutischer Stoff enthalten ist, die Spritze (8) vor dem Einsetzen in die zweite Zugangsöffnung (30) mit einer Flüssigkeit zum Auflösen des Stoffes gefüllt ist, nach dem Einsetzen der Spritze (8) in die zweite Zugangsöffnung (30) die Flüssigkeit aus der Spritze (8) in das Behältnis (18) überführt wird, um den pulverförmigen Stoff aufzulösen und so die medizinische oder pharmazeutische Flüssigkeit zu bilden, und anschließend die Flüssigkeit vorzugsweise wieder in dieselbe Spritze (8) gesaugt wird, um sie in die Zylinderampulle (7) zu überführen.

## Claims

1. A filling aid for filling a cylinder ampoule (7) with a medical or pharmaceutical liquid, wherein the filling aid in a common housing (4) has a first receptacle (24), which is designed to hold the cylinder ampoule (7) to be filled, and a second receptacle (26), which is designed to hold a container (18) containing a medical or pharmaceutical substance,
fixing means (32) are arranged in the first receptacle (24), which are designed and provided for detachably fixing a cylinder ampoule (7) in the first receptacle (24),
fixing means (36) are arranged in the second receptacle (26), which are designed and provided for fixing a container (18) containing a medical or pharmaceutical substance in the second receptacle (26),
a first access opening (28) is formed at the first receptacle (24), and arranged in such a way that it lies opposite an opening of a cylinder ampoule (7) when it has been placed in the first receptacle (24), and that the opening of the cylinder ampoule (7) is accessible from outside through the first access opening (28), and the housing (4) has a second access opening (30), which is formed on the second receptacle (26) and arranged in such a way that it lies opposite an opening of a container (18) when it has been placed in the second receptacle (26), and that an opening of the container is accessible through the second access opening (30), **characterized in that**
the filling aid has a closure element (6) that can be moved between a first and a second position, which in the first position closes the first access opening (28) and releases the second access opening (30), and in its second position closes the second access opening (30) and releases the first access opening (28).

2. The filling aid according to claim 1, in which a cylinder ampoule (7) is preassembled and detachably fixed in the first receptacle (24) with the fixing means (32), and can also be removed from the first receptacle (24).

3. The filling aid according to claim 1 or 2, in which the fixing means (36) arranged in the second receptacle (26) are designed and provided for detachably fixing a container (18) containing a medical or pharmaceutical substance in the form of a vial in the second receptacle (26).

4. The filling aid according to one of the preceding claims, in which a canula element (40) having a canula (44) and a Luer connection (42) is detachably held in the second receptacle (26), wherein the Luer connection (42) of the canula element (40) faces the second access opening (30) and the canula (44) is directed in such a way as to penetrate into a container (18) placed in the second receptacle (26).

5. The filling aid according to claim 4, in which the second access opening (30) is dimensioned in such a way that the canula element (40) can be removed through the second access opening (30).

6. The filling aid according to one of the preceding claims, in which at least one holding means (38) is arranged in the second receptacle, and detachably fixes the canula element (40), wherein the holding means (38) preferably generates a holding force in the longitudinal direction of the canula (44) that is less than a force transmissible by the Luer connection (42) in the longitudinal direction.

7. The filling aid according to one of the preceding claims, which has a syringe that is separate from the housing (4) and has a Luer cone (14), which can be engaged with the Luer connection (42) of the canula element (40).

8. The filling aid according to one of the preceding claims, in which the housing (4) has a third receptacle (64), which is designed for holding and fixing a syringe (8).

9. The filling aid according to one of the preceding claims, in which the first access opening (28) is dimensioned in such a way that a canula element (40), and preferably the canula element (40) defined in claims 4 to 8, can be inserted in the first receptacle (24) through the first access opening (28).

10. The filling aid according to claim 9, in which at least one safeguard (60) is arranged in the first receptacle (24) at the first access opening (28), and designed in such a way as to fix a canula element (40) inserted through the first access opening (28), preferably so that it cannot be removed again.

11. The filling aid according to claim 10, in which the at least one safeguard is designed as a latching element, and in particular as a latching tongue (60).

12. The filling aid according to one of the preceding claims, in which the closure element (6) has at least one detachable lock (50, 52), which detachably fixes the closure element (6) in its first and/or its second position, wherein the lock (50, 52) can preferably be detached onehandedly.

13. The filling aid according to one of the preceding claims, in which the first (28) and the second (30) access opening are arranged on the same side of the housing (4).

14. The filling aid according to one of the preceding claims, in which the first receptacle (24) with the first access opening (28) and the second receptacle (26) with the second access opening (30) are designed in such a way that the insertion directions (E1, E2) of the first and the second access opening (28, 30), along which a canula element (40) and/or a syringe (8) can be inserted into the access openings (28, 30), run parallel to each other.

15. A method for filling a cylinder ampoule (7) with a medical or pharmaceutical liquid using a filling aid according to one of the preceding claims, in which, sequentially, a container (18) with the medical or pharmaceutical liquid to be introduced into the cylinder ampoule (7) has been or is inserted into the second receptacle (26),
a syringe (8) is inserted into the second access opening (30),
the medical or pharmaceutical liquid is siphoned out of the container (18) into the syringe (8),
the syringe (8) is removed from the second access opening (30) and inserted into the first access opening (28), and
the medical or pharmaceutical liquid is transferred from the syringe (8) into a cylinder ampoule (7) arranged in the first receptacle (24).

16. The method according to claim 15, in which a powdery medical or pharmaceutical substance is initially contained in the container (18), and dissolved by adding a liquid, so as to generate the medical or pharmaceutical liquid in the container (18) in this way.

17. The method according to claim 16, in which a powdery medical or pharmaceutical substance is initially contained in the container (18), the syringe (8) is filled with a liquid for dissolving the substance prior to insertion into the second access opening (30), the liquid is transferred from the syringe (8) into the container (18) after insertion of the syringe (8) into the second access opening (30), so as to dissolve the powdery substance and thereby form the medical or pharmaceutical liquid, after which the liquid is preferably siphoned into the same syringe (8) again in order to transfer it into the cylinder ampoule (7).

## Revendications

1. Système d'aide au remplissage servant au remplissage d'une ampoule cylindrique (7) avec un liquide médical ou pharmaceutique, sachant que le système d'aide au remplissage comporte dans un boîtier (4) commun, un premier logement (24), lequel est constitué pour recevoir l'ampoule cylindrique (7) à remplir, et un deuxième logement (26), lequel est constitué pour recevoir un contenant (18) contenant une substance médicale ou pharmaceutique,
des moyens de fixation (32) sont disposés dans le premier logement (24), lesquels sont constitués et prévus pour fixer de façon amovible une ampoule cylindrique (7) dans le premier logement (24),
des moyens de fixation (36) sont disposés dans le deuxième logement (26), lesquels sont constitués et prévus pour fixer un contenant (18) contenant une substance médicale ou pharmaceutique dans le deuxième logement (26),
une première ouverture d'accès (28) est constituée sur le premier logement (24), laquelle est disposée de telle manière qu'elle est opposée à une ouverture d'une ampoule cylindrique (7), lorsqu'elle est insérée dans le premier logement (24) et que l'ouverture de l'ampoule cylindrique (7) est accessible de l'extérieur par la première ouverture d'accès (28), et
le boîtier (4) comporte une deuxième ouverture d'accès (30), laquelle est constituée sur le deuxième logement (26) et est disposée de telle manière qu'elle est opposée à une ouverture d'un contenant (18), lorsqu'il est inséré dans le deuxième logement (26) et qu'une ouverture du contenant est accessible par la deuxième ouverture d'accès (30), **caractérisé en ce que**
le système d'aide au remplissage comporte un élément de fermeture (6) mobile entre une première et une deuxième position, lequel ferme dans la première position la première ouverture d'accès (28) et libère la deuxième ouverture d'accès (30) et ferme dans sa deuxième position la deuxième ouverture d'accès (30) et libère la première ouverture d'accès (28).

2. Système d'aide au remplissage selon la revendication 1, pour lequel une ampoule cylindrique (7) est prémontée et est fixée de façon amovible dans le premier logement (24) avec les moyens de fixation (32) et peut être enlevée du premier logement (24).

3. Système d'aide au remplissage selon la revendication 1 ou 2, pour lequel les moyens de fixation (36) disposés dans le deuxième logement (26) sont constitués et prévus pour fixer de façon amovible un contenant (18) contenant une substance médicale ou pharmaceutique sous la forme d'un flacon dans le deuxième logement (26).

4. Système d'aide au remplissage selon l'une quelconque des revendications précédentes, pour lequel un élément de canule (40), lequel comporte une canule (44) et un embout Luer (42), est maintenu de façon amovible dans le deuxième logement (26), sachant que l'embout Luer (42) de l'élément de canule (40) est tourné vers la deuxième ouverture d'accès (30) et la canule (44) est dirigée de telle manière qu'elle pénètre dans un contenant (18) inséré dans le deuxième logement (26).

5. Système d'aide au remplissage selon la revendication 4, pour lequel la deuxième ouverture d'accès (30) est dimensionnée de telle sorte que l'élément de canule (40) peut être enlevé par la deuxième ouverture d'accès (30).

6. Système d'aide au remplissage selon l'une quelconque des revendications précédentes, pour lequel un moyen de retenue (38) est disposé dans le deuxième logement, lequel fixe de façon amovible l'élément de canule (40), sachant que le moyen de retenue (38) produit de préférence une force de retenue dans la direction longitudinale de la canule (44), laquelle est plus faible qu'une force transmissible par l'embout Luer (42) dans la direction longitudinale.

7. Système d'aide au remplissage selon l'une quelconque des revendications précédentes, lequel comporte une seringue séparée du boîtier (4) avec un cône Luer (14), lequel peut être mis en prise avec l'embout Luer (42) de l'élément de canule (40).

8. Système d'aide au remplissage selon l'une quelconque des revendications précédentes, pour lequel le boîtier (4) comporte un troisième logement (64), lequel est constitué pour recevoir et fixer une seringue (8).

9. Système d'aide au remplissage selon l'une quelconque des revendications précédentes, pour lequel la première ouverture d'accès (28) est dimensionnée de telle sorte qu'un élément de canule (40), et de préférence l'élément de canule (40) défini dans les revendications 4 à 8, peut être inséré dans le premier logement (24) par la première ouverture d'accès (28).

10. Système d'aide au remplissage selon la revendication 9, pour lequel au moins un moyen de sécurisation (60) est disposé dans le premier logement (24) sur la première ouverture d'accès (28), lequel est constitué de telle manière qu'il fixe, et de préférence fixe ne pouvant être à nouveau enlevé, un élément de canule (40) inséré par la première ouverture d'accès (28).

11. Système d'aide au remplissage selon la revendication 10, pour lequel au moins un moyen de sécurisation est constitué sous la forme d'un élément d'enclenchement et en particulier sous la forme d'une languette d'enclenchement (60).

12. Système d'aide au remplissage selon l'une quelconque des revendications précédentes, pour lequel l'élément de fermeture (6) comporte au moins un dispositif de blocage amovible (50, 52), lequel fixe de façon amovible l'élément de fermeture (6) dans sa première et/ou sa deuxième position, sachant que le dispositif de blocage (50, 52) peut être débloqué de préférence avec une seule main.

13. Système d'aide au remplissage selon l'une quelconque des revendications précédentes, pour lequel la première (28) et la deuxième (30) ouverture d'accès sont disposées sur le même côté du boîtier (4).

14. Système d'aide au remplissage selon l'une quelconque des revendications précédentes, pour lequel le premier logement (24) avec la première ouverture d'accès (28) et le deuxième logement (26) avec la deuxième ouverture d'accès (30) sont constitués de telle manière que les sens d'insertion (E1, E2) de la première et de la deuxième ouverture d'accès (28, 30), le long desquelles un élément de canule (40) et/ou une seringue (8) peut être inséré dans les ouvertures d'accès (28, 30), passent parallèlement l'un à l'autre.

15. Procédé de remplissage d'une ampoule cylindrique (7) avec un liquide médical ou pharmaceutique en utilisant un système d'aide au remplissage selon l'une quelconque des revendications précédentes, pour lequel successivement un contenant (18) avec le liquide médical ou pharmaceutique à introduire dans l'ampoule cylindrique (7) est inséré ou sera inséré dans le deuxième logement (26),
une seringue (8) sera insérée dans la deuxième ouverture d'accès (30), le liquide médical ou pharmaceutique sera aspiré du contenant (18) dans la seringue (8),
la seringue (8) est enlevée de la deuxième ouverture d'accès (30) et insérée dans la première ouverture d'accès (28), et
le liquide médical ou pharmaceutique sera transféré de la seringue (8) dans une ampoule cylindrique (7) disposée dans le premier logement (24).

16. Procédé selon la revendication 15, pour lequel une substance pulvérulente médicale ou pharmaceutique est d'abord contenue dans le contenant (18), laquelle sera dissoute par addition d'un liquide pour produire ainsi le liquide médical ou pharmaceutique dans le contenant (18).

17. Procédé selon la revendication 16, pour lequel une substance pulvérulente médicale ou pharmaceutique est d'abord contenue dans le contenant (18), la seringue (8) est remplie avec un liquide pour dissoudre la substance avant l'insertion dans la deuxième ouverture d'accès (30), le liquide est transféré de la seringue (8) dans le contenant (18) après l'insertion de la seringue (8) dans la deuxième ouverture d'accès (30) pour dissoudre la substance pulvérulente et former ainsi le liquide médical ou pharmaceutique et transférer ensuite le liquide de préférence à nouveau dans cette même seringue (8) pour le transférer dans l'ampoule cylindrique (7).
